# EUROPEAN PATENT SPECIFICATION

(11) **EP 2 566 451 B1**
(45) Date of publication and mention of the grant of the patent: **10.09.2014**
(21) Application number: 11724820.3
(22) Date of filing: 02.05.2011
(51) Int. Cl.: A61K 9/00, A61K 31/546

(54) **PHARMACEUTICAL COMPOSITIONS COMPRISING CEFDITOREN PIVOXIL**
PHARMAZEUTISCHE ZUBEREITUNG ENTHALTEND CEFDITOREN PIVOXIL
COMPOSITION PHARMACEUTIQUE CONTENTANT DE CEFDITOREN PIVOXIL

(30) Priority: 04.05.2010 TR 201003548
(43) Date of publication of application: 13.03.2013
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2011/000125
(87) International publication number: WO 2011/139251

(56) References cited:
- WO-A1-2004/004737
- WO-A1-2005/055986
- CN-A- 1 846 702
- JP-A- 2004 043 475
- JP-A- 2007 106 684

## Description

The present invention relates to water dispersible forms comprising cefditoren pivoxil and/or pharmaceutically acceptable salts, hydrates, solvates, esters, amorphous and crystal forms and/or a combination thereof.

### Background of the Invention:

Cefditoren pivoxil is a third generation cephalosporin, which has the chemical name (6R,7R)-pivaloyloxymethyl 7-[(Z)-2-(2-aminothiazol-4-yl)-2-(methoxyimino)acetamido]-3 -[(Z)-2-(4-methylthiazol-5-yl)vinyl]-8-oxo-5-thia-1-azabicyclo[4.2.0]oct-2-ene-2-carboxylate, and it was first disclosed in the patent numbered EP0175610. Cefditoren pivoxil, the chemical structure of which is shown in formula 1, is indicated for the treatment of the infections caused by gram positive and gram negative bacteria.

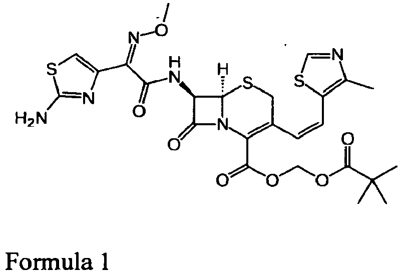

Cefditoren pivoxil is the ester form of the antibiotic known as cefditoren. In order to increase the absorption of cefditoren in the body, cefditoren pivoxil which is a form of pivaloyloxy methyl ester was produced. However, this caused that the substance, which is obtained, has a low water solubility.

Cefditoren pivoxil is available in oral dosage forms of 200 and 400 mg on the market. When the tablet forms comprising 200 and 400 mg active agent in a single dose are formulated with excipients, they become quite large in size and this made the use of this dosage form inconvenient in patients with dysphagia, particularly in pediatric and geriatric patients. The suspension forms which have been alternatively developed to overcome said problems are not preferred since they have the potential of high and/or uncontrolled dose intake and are not physically and chemically stable. Alternatively, the use of reliable and user-friendly water dispersible forms is suggested.

Cefditoren pivoxil has a low water solubility which is approximately less than 1 mg/ml. Accordingly, the formulations comprising cefditoren pivoxil as an active agent cannot dissolve easily and disperse quickly in water. Due to these problems, sufficient amount of cefditoren active agent can not be absorbed and thus its bioavailability decreases.

As it is seen, new formulations comprising cefditoren pivoxil are required to be developed in order to provide new dosage forms which are easily swallowed by pediatric and geriatric patients, which are easily dissolved and which are quickly dispersed in water during their usages.

The inventors have surprisingly found that the present problems in the prior art can be solved by the water dispersible powder, tablet and granule formulation prepared according to the subject of the present invention.

### Description of the Invention:

The present invention relates to the water dispersible powder, tablet and granules comprising cefditoren pivoxil and/or pharmaceutically acceptable salts, hydrates, solvates, esters, amorphous and crystal forms of cefditoren pivoxil and/or combination thereof; their formulations and the procedures for their preparation. Surprisingly, it has been found that the water dispersible powder, tablet and granule formulations comprising cefditoren pivoxil in which at least 65% of diluent is used and diluent: cefditoren pivoxil ratio is in the range of 15:1 and 1:1, dissolve in water entirely and disperse in 2-4 minutes.

Accordingly, pharmaceutical formulations in the form of water dispersible powder, tablet or granule pertaining to the present invention are convenient for the patients with dysphagia, particularly in pediatric and geriatric patients. Moreover, the absorption and bioavailability of cefditoren pivoxil are found to be increased due to achieving a high solubility and rapid dissolution rate.

The term "water dispersible powder, tablet and granule" present in the text comprises effervescent tablets, effervescent granules, effervescent powders, water dispersible tablets, water dispersible powders, water dispersible granules, water soluble tablets, water soluble powders and water soluble granules.

Accordingly, the first aspect of the present invention is the water dispersible formulations formulated in powder, tablet or granule form comprising cefditoren pivoxil in which at least 65% diluent is used and diluent: cefditoren pivoxil ratio is in the range of 15:1 and 1:1.

According to another aspect, diluent: cefditoren pivoxil ratio is in the range of 15:1 to 1:1, preferably in the range of 12:1 to 4:1, most preferably in the range of 10:1 to 5:1.

According to this, another aspect of the present invention is the water dispersible formulations formulated in powder, tablet or granule forms comprising cefditoren pivoxil in which at least 65% diluent is used and diluent: cefditoren pivoxil ratio is in the range of 15:1 to 1:1, preferably in the range of 12:1 to 4:1, most preferably in the range of 10:1 to 5:1.

Cefditoren pivoxil, which is the subject of the present invention, can be present in the form of its solvates, hydrates, esters, enantiomers, racemates, organic salts, inorganic salts, polymorphs, crystal and amorphous forms or in free form and/or a combination thereof in the water dispersible powder, tablet and granule formulations of the present invention. Preferably cefditoren pivoxil is in crystal or amorphous form, more preferably cefditoren pivoxil is in amorphous form.

The diluent used in water dispersible powder, tablet and granule formulations pertaining to the present invention can be selected from, but not limited to, a group comprising calcium carbonate, calcium sulphate, dibasic calcium phosphate, tribasic calcium phosphate, calcium phosphate, calcium sulphate, microcrystalline cellulose, lactose, magnesium carbonate, magnesium oxide, maltodextrin, maltose, mannitol, sodium chloride, sorbitol, starch and xylitol or combinations thereof. Preferably sorbitol is used as diluent in the water dispersible powder, tablet and granule formulations pertaining to the present invention.

According to this, another aspect of the present invention is the water dispersible formulations formulated in powder, tablet or granule forms comprising cefditoren pivoxil in which at least 65% sorbitol is used and sorbitol: cefditoren ratio is in the range of 15:1 and 1:1 by weight.

Various excipients selected from, but not limited to, a group comprising binders, lubricants, humectants, disintegrants, diluents, basic agents, acidic agents, sweeteners, taste regulating agents and optionally an effervescent couple can be used in the water dispersible formulation in addition to cefditoren pivoxil.

The binder that can be used in the water dispersible powder, tablet and granule formulations pertaining to the present invention can be selected from, but not limited to, a group comprising ethyl cellulose, gelatine, hydroxyethyl cellulose, hydroxymethyl cellulose, hydroxypropyl cellulose, hypromellose, magnesium aluminum silicate, methyl cellulose, povidone or combinations thereof.

The lubricant that can be used in the water dispersible powder, tablet and granule formulations pertaining to the present invention can be selected from, but not limited to, a group comprising calcium stearate, magnesium stearate, polyethylene glycol, PEG 6000, polyvinyl alcohol, potassium benzoate, sodium benzoate.

The disintegrant that can be used in water dispersible powder, tablet and granule formulations pertaining to the present invention can be selected from, but not limited to, a group comprising carboxymethyl cellulose calcium, carboxymethyl cellulose sodium, microcrystalline cellulose, silicon dioxide, croscarmellose sodium, crospovidone, hydroxypropyl cellulose, methyl cellulose, povidone, magnesium aluminum silicate and starch or combinations thereof.

The taste regulating agent and/or sweetener that can be used in water dispersible powder, tablet and granule formulations pertaining to the present invention can be selected from, but not limited to, a group comprising acesulfame, aspartame, dextrose, fructose, glucose, lactitol, maltitol, maltose, sorbitol, saccharine, saccharine sodium, sodium cyclamate, sucralose, sodium chloride, potassium chloride, sucrose, xylitol or combinations thereof. Preferably, sucrose, aspartame, sodium chloride or a combination thereof is used in the formulations according to the present invention. Preferably, aspartame is used as the sweetener in the water dispersible powder, tablet and granule formulations pertaining to the present invention.

Cefditoren pivoxil is a molecule which has a bitter taste and it is so difficult to eliminate this unpleasant taste in the pharmaceutical formulations. The inventors have found that the problem resulting from the bitter taste of cefditoren pivoxil is eliminated in the pharmaceutical formulations in which cefditoren pivoxil: sweetener ratio is in the range of 15:1 and 5:1, preferably 12:1 and 6:1 and the taste of water dispersible compositions comprising cefditoren pivoxil is found to be pleasant.

According to this, another aspect of the present invention is the water dispersible cefditoren pivoxil formulations in which cefditoren pivoxil: sweetener ratio is in the range of 15:1 and 5:1, preferably 12:1 and 6:1.

Effervescent acid that can optionally be used in water dispersible powder, tablet and granule formulations pertaining to the present invention can be selected from organic acids such as citric acid, tartaric acid, malic acid, fumaric acid, ascorbic acid, adipic acid and succinic acid.

Effervescent base that can optionally be used in water dispersible powder, tablet and granule formulations pertaining to the present invention can be selected from basic agents such as sodium hydrogen carbonate, sodium carbonate, potassium carbonate, potassium bicarbonate, sodium glycine carbonate, lysine carbonate, arginine carbonate and calcium carbonate.

In the water dispersible powder, tablet and granule formulation of the present invention, cefditoren pivoxil or its pharmaceutically acceptable salts, hydrates, solvates or combinations thereof can be used.

The water dispersible powder, tablet and granule formulation of the present invention can comprise 5-25% cefditoren pivoxil; 0-15% binder; 0-5% lubricant; 0-10% humectant, 0-20% disintegrant; 65-90% diluent; 0-15% basic agent; 0-20% acidic agent; 0,5-5% sweetener and/or taste regulating agent; 0.2-6% coloring and/or flavoring agent and 0-80% effervescent couple with respect to the total weight of unit dose.

In another aspect, the present invention relates to the processes which can be used for the preparation of water dispersible powder, tablet and granule formulations comprising pharmaceutically acceptable excipients in addition to cefditoren pivoxil as the active agent.

The pharmaceutical composition of the present invention can be prepared through a process which is comprised of dry blending or dry granulation or wet granulation of the components or a combination thereof and generally known standard techniques and manufacture procedures in technology.

In another aspect, the present invention relates to the preparation of a medicament comprising cefditoren pivoxil so as to be used in the treatment of infections caused by gram positive and gram negative bacteria.

According to another aspect of the present invention, the pharmaceutical composition prepared according to the present invention is used in the manufacture of a medicament so as to be used in upper respiratory infections such as ear, nose, throat, otitis media, sinusitis, tonsillitis, pharyngitis; lower respiratory tract infections such as pyelonephritis, cystitis and urethritis; skin or soft tissue infections such as froncle, pyoderma, impetigo; in the treatment and prophylaxis of gonorrhea and lyme diseases.

### EXAMPLE 1: Formulation and process for the preparation of water dispersible granules

| | % of amount present in unit dose |
|---|---|
| Cefditoren Pivoxil | 7.5% |
| Diluent | 75.0% |
| Sweetener | 2.0% |
| Binder | 9.0% |
| Flavoring agent | 5.0% |
| Coloring agent | 1.5% |

According to this, the process in scope of the present invention comprises granulating a mixture comprising cefditoren pivoxil and other excipients with the granulation solution comprising the coloring agent and the diluent through conventional wet and/or dry granulation methods in the prior art, then sieving the mixture; adding a flavoring agent and/or agents into the obtained granules, and optionally compressing the obtained mixture in tablet compressing machine.

## Claims

1. A water dispersible formulation formulated in tablet or granule form comprising cefditoren pivoxil **characterized in that** at least 65% diluent is used with respect to the total weight of the unit dosage and diluent: cefditoren ratio is in the range of 15:1 and 1:1.

2. The pharmaceutical composition according to claim 1, wherein diluent: cefditoren ratio is in the range of 12:1 to 4:1.

3. The pharmaceutical composition according to claim 2, wherein diluent: cefditoren ratio is in the range of 10:1 to 5:1.

4. The pharmaceutical composition according to claims 1-3, wherein diluent is selected from a group comprising calcium carbonate, dibasic calcium phosphate, tribasic calcium phosphate, calcium phosphate, calcium sulphate, microcrystalline cellulose, lactose, magnesium carbonate, magnesium oxide, maltodextrin, maltose, mannitol, sodium chloride, sorbitol, starch and xylitol or combinations thereof.

5. The pharmaceutical composition according to claim 5, wherein sorbitol is used as diluent.

6. The pharmaceutical composition according to claim 1 - 5, wherein said composition comprises one or more binders, lubricant, humectant, disintegrant, basic agent, acidic agent, taste regulating agent and optionally effervescent couple comprising of an effervescent acid and an effervescent base in addition to cefditoren pivoxil that is used as the active agent.

7. The formulation according to claim 6, wherein taste regulating agent and/or sweetener is selected from a group comprising acesulfame, aspartame, dextrose, fructose, glucose, lactitol, maltitol, sorbitol, saccharine sodium, sodium cyclamate, sucralose, sodium chloride, potassium chloride, sucrose, xylitol or combinations thereof.

8. The formulation according to claim 7, wherein taste regulating agent and/or sweetener is selected from sucrose, aspartame, sodium chloride or a combination thereof.

9. The formulation according to claims 7-8, wherein aspartame is used as taste regulating agent and/or sweetener.

10. The formulation according to claims 1-9, wherein cefditoren pivoxil: sweetener ratio is in the range of 15:1 and 5:1.

11. The formulation according to claim 10, wherein cefditoren pivoxil: sweetener ratio is in the range of 12:1 and 6:1.

12. The formulation according to claim 6, wherein effervescent acid that is optionally used is selected from organic acids such as citric acid, tartaric acid, malic acid, fumaric acid, ascorbic acid, adipic acid and succinic acid.

13. The formulation according to claim 6, wherein effervescent base that is optionally used is selected from basic agents such as sodium hydrogen carbonate, sodium carbonate, potassium carbonate, potassium bicarbonate, sodium glycine carbonate, lysine carbonate, arginine carbonate and calcium carbonate.

14. The pharmaceutical composition according to claims 1-13, wherein said composition comprises 5-25 % cefditoren; 0-15% binder; 0-5% lubricant; 0-10% humectant, 0-20% disintegrant; 65-90% diluent; 0-15% basic agent; 0-20% acidic agent; 0,5-5% sweetener and/or taste regulating agent; 0.2-6% coloring and/or flavoring agent and 0-80% effervescent couple with respect to the total amount of unit dose.

15. The method for the preparation of a pharmaceutical composition according to claims 1-14, wherein said method comprises a process of dry blending or dry granulation or wet granulation of the components or a combination thereof, and generally known standard techniques and manufacture procedures in technology.

## Patentansprüche

1. Eine wasserdispergierbare Formulierung, die in Tabletten-oder Granulatform formuliert wird, die Pivoxilcefditoren enthält und **dadurch gekennzeichnet** wird, dass mindestens 65% vom Verdünnungsmittel in Bezug auf das Gesamtgewicht der Dosierungseinheit und Verdünnungsmittel verwendet werden: das Verhältnis von Cefditoren ist im Bereich zwischen 15:1 und 1:1.

2. Während es die pharmazeutische Zusammensetzung gemäß Anspruch 1 ist, ist das Verhältnis von Verdünnungsmittel: Cefditoren im Bereich zwischen 12:1 und 4:1.

3. Während es die pharmazeutische Zusammensetzung gemäß Anspruch 2 ist, ist das Verhältnis von Verdünnungsmittel: Cefditoren im Bereich zwischen 10:1 und 5:1.

4. Während es die pharmazeutische Zusammensetzung gemäß den Ansprüchen 1-3 ist, wird das Verdünnungsmittel aus einer Gruppe ausgewählt, die Calciumcarbonat, zweibasisches Calciumphosphat , dreibasisches Calciumphosphat, Calciumphosphat, Calciumsulfat, mikrokristalline Cellulose, Lactose, Magnesiumcarbonat, Magnesiumoxid, Maltodextrin, Maltose, Mannit, Natriumchlorid, Sorbitol, Stärke und Xylitol oder Kombinationen davon beinhaltet.

5. Während es die pharmazeutische Zusammensetzung gemäß Anspruch 5 ist, wird Sorbitol als Verdünnungsmittel verwendet.

6. Während es die pharmazeutische Zusammensetzung gemäß den Ansprüchen 1 - 5 ist, enthält die erwähnten Zusammensetzung ein oder mehrere Bindemittel, Gleitmittel, Feuchthaltemittel, Sprengmittel, Basismittel, saures Mittel, Geschmacksregulierendes Mittel und gegebenenfalls Brausepaar, das aus einer brausenden Säure und einer Brausegrundlage neben Pivoxilcefditoren besteht, das als Wirkstoff verwendet wird.

7. Während es die Formulierung gemäß Anspruch 6 ist, werden Geschmacksregulierendes Mittel und / oder Süßstoff aus einer Gruppe ausgewählt, die Acesulfam, Aspartam, Dextrose, Fructose, Glucose, Lactitol, Maltit, Sorbitol, Saccharin-Natrium, Natriumcyclamat, Sucralose, Natriumchlorid, Kaliumchlorid , Saccharose, Xylitol oder Kombinationen davon beinhaltet.

8. Während es die Formulierung gemäß Anspruch 7 ist, werden Geschmacksregulierendes Mittel und / oder Süßstoff aus Saccharose, Aspartam, Natriumchlorid oder einer Kombination davon ausgewählt.

9. Während es die Formulierung gemäß den Ansprüchen 7-8 ist, wird Aspartam als Geschmacksregulierungs- und / oder Süßungsmittel verwendet.

10. Während es die Formulierung gemäß den Ansprüchen 1-9 ist, ist das Verhältnis von Pivoxilcefditoren: Süßungsmittel im Bereich zwischen 15:1 und 5:1.

11. Während es die Formulierung gemäß Anspruch 10 ist, ist das Verhältnis von Pivoxilcefditoren: Süßungsmittel im Bereich zwischen 12:1 und 6:1.

12. Während es die Formulierung gemäß Anspruch 6 ist, wird die gegebenenfalls verwendeten Brausesäure aus organischen Säuren wie Zitronensäure, Weinsäure , Äpfelsäure, Fumarsäure, Ascorbinsäure, Adipinsäure und Bernsteinsäure ausgewählt.

13. Während es die Formulierung gemäß Anspruch 6 ist, wird die gegebenenfalls verwendeten Brausegrundlage aus basischen Mittel wie Natriumhydrogencarbonat, Natriumcarbonat, Kaliumcarbonat, Kaliumbicarbonat Natrium-Glycin-Carbonat, Lysin- Carbonat, Arginin und Calciumcarbonat ausgewählt.

14. Während es die pharmazeutische Zusammensetzung nach den Ansprüchen 1-13 ist, beinhaltet die erwähnten Zusammensetzung 5-25% von Cefditoren; 0-15% von Bindemittel; 0-5% von Schmiermittel; 0-10% von Feuchthaltemittel, 0-20% von Sprengmittel; 65-90% von Verdünnungsmittel; 0-15% von Grundmittel ; 0-20% vom sauren Mittel; 0,5-5% von Süßstoff und / oder Geschmacksregulierendes Mittel; 0,2-6% von Farbstoffe und / oder Aromamittel und 0-80% von Brausepaar in Bezug auf die Gesamtmenge der Dosierungseinheit.

15. Während es das Verfahren zur Herstellung einer pharmazeutischen Zusammensetzung gemäß den Ansprüchen 1 - 14 ist, beinhaltet das erwähnte Verfahren einen Prozess vom Trockenmischen oder Trockengranulation oder Feuchtgranulation der Bestandteile oder eine Kombination davon und allgemein bekannte Standardtechniken und Herstellungsverfahren in der Technik.

## Revendications

1. Une formulation dispersible dans l'eau formulée sous la forme d'un comprimé ou un granule comprenant cefditoren pivoxil **caractérisée en ce qu'**au moins 65% diluant est utilisé par rapport au poids total de la dose unitaire et que la proportion de diluant : cefditoren est de 15:1 à 1 :1.

2. La composition pharmaceutique selon la revendication 1, dans laquelle la proportion de diluant : cefditoren est de 12 :1 à 4 :1.

3. La composition pharmaceutique selon la revendication 2, dans laquelle la proportion de diluant : cefditoren est de 10 :1 à 5 :1.

4. La composition pharmaceutique selon les revendications 1-3, dans laquelle le diluant est choisi d'un groupe comprenant carbonate de calcium, phosphate de calcium dibasique, phosphate de calcium tribasique, phosphate de calcium, sulfate de calcium, cellulose microcristalline, lactose, carbonate de magnésium, oxyde de magnésium, maltodextrine, maltose, mannitol, chlorure de sodium, sorbitol, amidon et xylitol ou combinaisons de ceux-ci.

5. La composition pharmaceutique selon la revendication 5, dans laquelle sorbitol est utilisé comme le diluant.

6. La composition pharmaceutique selon les revendications 1-5, dans laquelle ladite composition comprend un ou plusieurs liant(s), lubrifiant, humectant, désintégrant, agent basique, agent acide, agent régulateur de goût et facultativement un couple effervescent comprenant un acide effervescent et une base effervescente en plus de cefditoren pivoxil qui est utilisé comme l'agent actif.

7. La formulation selon la revendication 6, dans laquelle l'agent régulateur de goût et/ou l'édulcorant est choisi dans un groupe comprenant acésulfame, aspartame, dextrose, fructose, glucose, lactitol, maltitol, sorbitol, saccharine de sodium, cyclamate de sodium, sucralose, chlorure de sodium, chlorure de potassium, sucrose, xylitol ou combinaisons de ceux-ci.

8. La formulation selon la revendication 7, dans laquelle l'agent régulateur de goût et/ou l'édulcorant est choisi parmi sucrose, aspartame, chlorure de sodium ou une combinaison de ceux-ci.

9. La formulation selon les revendications 7-8, dans laquelle aspartame est utilisé comme l'agent régulateur de goût et/ou l'édulcorant.

10. La formulation selon les revendications 1-9, dans laquelle la proportion de cefditoren pivoxil : édulcorant est de 15 :1 à 5 :1.

11. La formulation selon la revendication 10, dans laquelle la proportion de cefditoren pivoxil : édulcorant est de 12 :1 à 6 :1.

12. La formulation selon la revendication 6, dans laquelle l'acide effervescent facultativement utilisé est choisi parmi les acides organiques tels que acide citrique, acide tartrique, acide malique, acide fumarique, acide ascorbique, acide adipique et acide succinique.

13. La formulation selon la revendication 6, dans laquelle la base effervescente facultativement utilisée est choisi parmi les agents basiques tels que carbonate de sodium hydrogène, carbonate de sodium, carbonate de potassium, bicarbonate de potassium, carbonate de sodium glycine, carbonate de lysine, carbonate d'arginine et carbonate de calcium.

14. La composition pharmaceutique selon les revendications 1-13, dans laquelle ladite composition comprend 5-25% cefditoren ; 0-15% liant ; 0-5% lubrifiant ; 0-10% humectant; 0-20% désintégrant ; 65-90% diluant; 0-15% agent basique ; 0-20% agent acide; 0,5-5% édulcorant et/ou l'agent régulateur de goût; 0,2-6% agent colorant et/ou aromatisant et 0-80% couple effervescent par rapport au poids total de la dose unitaire.

15. La méthode de préparation d'une composition pharmaceutique selon les revendications 1-14, dans laquelle ladite méthode comprend un procédé de mélange à sec ou granulation sèche ou granulation humide des components ou une combinaison de ceux-ci, et les techniques standards et les procédés de fabrication généralement connus dans la technique.
